# EUROPEAN PATENT APPLICATION

(11) **EP 4 176 794 A1**
(43) Date of publication of application: **10.05.2023**
(21) Application number: 20943972.8
(22) Date of filing: 06.07.2020
(51) Int. Cl.: A61B 1/24

(54) **PROCESSING DEVICE, PROCESSING PROGRAM, PROCESSING METHOD, AND PROCESSING SYSTEM**

(71) Applicant: Aillis Inc., Chiyoda-ku Tokyo 100-0006 (JP)
(72) Inventor: FUKUDA Atsushi, Tokyo 100-0006 (JP); SODE Masashi, Tokyo 100-0006 (JP); CAP Huu Quan, Tokyo 100-0006 (JP); YASUMI Takashi, Tokyo 100-0006 (JP)
(74) Representative: Reeve, Nicholas Edward
(86) International application number: PCT/JP2020/026479
(87) International publication number: WO 2022/009285

(57) **Abstract**

To provide a processing device or the like that is suitable for processing an image obtained by photographing the inside of an oral cavity to be used for intraoral diagnosis. Provided is a processing device comprising: an input/output interface which is communicably connected to a camera for capturing an image, of a subject, at least including an oral cavity, and which is configured to receive an input of the image captured by the camera; a memory which is configured to store the image received by the input/output interface in addition to computer readable instructions; and a processor which is configured to, through execution of the computer readable instructions stored in the memory, specify a prescribed area in the subject included in the image on the basis of the image stored in the memory, and to process the image in such a manner as to render the specified prescribed area identifiable.

## Description

### Technical Field

The present disclosure relates to a processing device, a processing program, a processing method, and a processing system for processing an image of a subject that is captured by a camera.

### Background Art

From the related art, it has been known that a medical doctor observes a change in the state of an oral cavity of a target, and for example, makes a diagnosis such as a viral cold. Here, it is known that a lymphoid follicle that appears in the deepest portion of a pharynx positioned in the oral cavity has a pattern specific to influenza. The lymphoid follicle having such a specific pattern is referred to as an influenza follicle, is a characteristic sign for the influenza, and appears in onset. However, such a pharynx portion is diagnosed by direct inspection of the medical doctor, and is not diagnosed by using an image.

On the other hand, an endoscope for capturing a deep portion in the body such as an abdominal cavity, which includes an imaging optical system on the head, has been known (Patent Literature 1). Since such an endoscope is affected by the contamination or the clouding of a capturing window, while cleaning liquid supply means supplies a cleaning liquid, vibration means vibrates at an intensity at which the contamination of a vibrator can be mixed with the cleaning liquid, and then, the vibration at an intensity at which the vibrator can be mixed with the cleaning liquid is switched to a vibration at an intensity weaker than the intensity described above, and the surface of a transparent member is dried, and thus, the influence of the contamination or the clouding of the capturing window is removed.

### Citation List

### Patent Literature

Patent Literature 1: JP 2009-189496 A

### Summary of Invention

### Technical Problem

It is difficult to use a massive device such as an endoscope in the oral cavity such as a pharynx or a tonsil in which a medical opinion is determined routinely, and a diagnosis is made in a short period of time. In addition, the influence of the clouding on the capturing of the oral cavity (in particular, the pharynx portion) where the external air and the air in the body flow in and out at a high frequency due to aspiration has been extremely serious. Further, in the oral cavity, various regions with an active movement, such as teeth or a tongue, are close to each other, and it is difficult to stably capture a sharp image. Therefore, on the basis of the technology as described above, an object of the present disclosure is to provide a processing device, a processing program, a processing method, and a processing system that are suitable for processing an image obtained by capturing an oral cavity to be used in a diagnosis of the oral cavity.

### Solution to Problem

According to one aspect of the present disclosure, a "processing device, including: an input/output interface connected to a camera capturing an image of a subject including at least an oral cavity such that communication is available and configured to receive input of the image captured by the camera; a memory configured to store the image received by the input/output interface, In addition to computer readable instructions; and a processor configured to execute the computer readable instructions stored in the memory to specify a predetermined region in the subject included in the image, on the basis of the image stored in the memory, and to process the image such that the specified predetermined region is identifiable" is provided.

According to one aspect of the present disclosure, a "processing device, including: an input/output interface connected to a camera capturing an image of a subject including at least an oral cavity such that communication is available and configured to receive input of the image captured by the camera; a memory configured to store the image received by the input/output interface, in addition to computer readable instructions; and a processor configured to execute the computer readable instructions stored in the memory to perform processing of removing clouding included in the image from the image stored in the memory" is provided.

According to one aspect of the present disclosure, a "processing device, including: an input/output interface connected to a camera capturing an image of a subject including at least an oral cavity such that communication is available and configured to receive input of the image captured by the camera; a memory configured to store the image received by the input/output interface, in addition to computer readable instructions; and a processor configured to execute the computer readable instructions stored in the memory to perform super-resolution processing with respect to the image for increasing a resolution of the image stored in the memory" is provided.

According to one aspect of the present disclosure, a "processing program allowing a computer, which includes: an input/output interface connected to a camera capturing an image of a subject including at least an oral cavity such that communication is available; and a memory configured to store the image, to function as a processor configured to receive input of the image captured by the camera, to store the image received by the input/output interface, to specify a predetermined region in the subject included in the image, on the basis of the image stored in the memory, and to process the image such that the specified predetermined region is identifiable" is provided.

According to one aspect of the present disclosure, a "processing method performed by executing computer readable instructions with a processor in a computer comprising an input/output interface connected to a camera capturing an image of a subject including at least an oral cavity such that communication is available; and a memory configured to store the image, in addition to the computer readable instructions, the method comprising the steps of: receiving input of the image captured by the camera; a step of storing the image received by the input/output interface; specifying a predetermined region in the subject included in the image, on the basis of the image stored in the memory; and processing the image such that the specified predetermined region is identifiable" is provided.

According to one aspect of the present disclosure, a "processing system, including: a capturing device configured to capture an image of a subject including at least an oral cavity; and the processing device described above" is provided.

### Advantageous Effects of Invention

According to the present disclosure, it is possible to provide the processing device, the processing program, the processing method, and the processing system that are suitable for processing the image obtained by capturing the oral cavity to be used in the diagnosis of the oral cavity.

Note that, the effect described above is merely an example for explanatory convenience, and is not limited. In addition to the effect described above or instead of the effect described above, any effect that is described in the present disclosure or an effect that is obvious for a person skilled in the art can also be obtained.

### Brief Description of Drawings

Fig. 1 is a diagram illustrating a usage state of a processing system 1 according to one embodiment of the present disclosure.
Fig. 2 is a schematic view of the processing system 1 according to one embodiment of the present disclosure.
Fig. 3 is a block diagram illustrating a configuration of the processing system 1 according to one embodiment of the present disclosure.
Fig. 4 is a schematic view illustrating a configuration of a top surface of a capturing device 200 according to one embodiment of the present disclosure.
Fig. 5 is a schematic view illustrating a configuration of a sectional surface of the capturing device 200 according to one embodiment of the present disclosure.
Fig. 6 is a diagram illustrating a processing flow that is executed in a processing device 100 according to one embodiment of the present disclosure.
Fig. 7 is a diagram illustrating a processing flow that is executed in the processing device 100 according to one embodiment of the present disclosure.
Fig. 8 is a diagram illustrating a processing flow relevant to generation of a learned model according to one embodiment of the present disclosure.
Fig. 9 is a diagram illustrating a processing flow relevant to the generation of a learned model according to one embodiment of the present disclosure.
Fig. 10 is a diagram illustrating a processing flow relevant to the generation of a learned model according to one embodiment of the present disclosure.
Fig. 11 is a diagram illustrating an example of a screen that is displayed on the processing device 100 according to one embodiment of the present disclosure.
Fig. 12 is a diagram illustrating an example of the screen that is displayed on the processing device 100 according to one embodiment of the present disclosure.
Fig. 13 is a diagram illustrating an example of the screen that is displayed on the processing device 100 according to one embodiment of the present disclosure.
Fig. 14 is a diagram illustrating an example of the screen that is displayed on the processing device 100 according to one embodiment of the present disclosure.
Fig. 15 is a diagram illustrating an example of the screen that is displayed on the processing device 100 according to one embodiment of the present disclosure.
Fig. 16 is a diagram illustrating an example of the screen that is displayed on the processing device 100 according to one embodiment of the present disclosure.
Fig. 17 is a diagram illustrating an example of the screen that is displayed on the processing device 100 according to one embodiment of the present disclosure.
Fig. 18 is a diagram illustrating an example of the screen that is displayed on the processing device 100 according to one embodiment of the present disclosure.
Fig. 19 is a diagram illustrating an example of an image obtained by the processing device 100 according to one embodiment of the present disclosure.
Fig. 20 is a diagram illustrating an example of the image obtained by the processing device 100 according to one embodiment of the present disclosure.
Fig. 21 is a diagram illustrating an example of the image obtained by the processing device 100 according to one embodiment of the present disclosure.
Fig. 22 is a diagram illustrating an example of the image obtained by the processing device 100 according to one embodiment of the present disclosure.
Fig. 23 is a diagram illustrating an example of the image obtained by the processing device 100 according to one embodiment of the present disclosure.
Fig. 24 is a diagram illustrating an example of image output according to one embodiment of the present disclosure.
Fig. 25 is a block diagram illustrating a configuration of the processing system 1 according to another embodiment of the present disclosure.

### Description of Embodiments

Various embodiments of the present disclosure will be described with reference to the attached drawings. Note that, the same reference numerals will be applied to common constituents in the drawings.

### <First Embodiment>

### 1. Outline of Processing System 1

A processing system 1 according to the present disclosure is used for obtaining a subject image by mainly capturing the inside of an oral cavity of a test subject. In particular, the processing system 1 is used for capturing the periphery of the back of a larynx of the oral cavity, specifically, a pharynx. Therefore, hereinafter, a case where the processing system 1 according to the present disclosure is used for capturing the pharynx will be mainly described. Here, the pharynx is an example of a capturing part, and naturally, the processing system 1 according to the present disclosure can also be preferably used in another part such as a tonsil insofar as the part is in the oral cavity.

As an example, the processing system 1 according to the present disclosure is used for specifying a region included in an image obtained by capturing the subject including a pharynx region in the oral cavity of the target, from the image. The image captured by the processing system 1, in which the region is specified, for example, is used by a medical doctor to determine the possibility of incidence of influenza. Here, the possibility of incidence of the influenza is determined by examining the pharynx region or a tonsil region of the target, or by determining the presence or absence of a medical opinion such as a follicle in the pharynx region. Therefore, as an example, the processing system 1 is capable of specifying the pharynx, the tonsil, or a region in which the follicle is expressed, in the obtained image. Note that, the determination of the possibility of incidence of the influenza is an example, and naturally, any determination can also be preferably used insofar as the determination is for a disease in which a difference occurs in the medical opinion of the oral cavity by incidence. Examples of such a disease include a streptococcal infection, an adenovirus infection, an EB virus infection, a mycoplasma infection, arteriosclerosis, and the like.

Note that, in the present disclosure, even though terms such as the "determination" and a "diagnosis" are used for the disease, the terms do not necessarily indicate definitive determination or a definitive diagnosis of the medical doctor. For example, by a capturing target oneself or a user other than the medical doctor using the processing system 1 of the present disclosure, naturally, the determination or the diagnosis can also be performed by a processing device 100 included in the processing system 1.

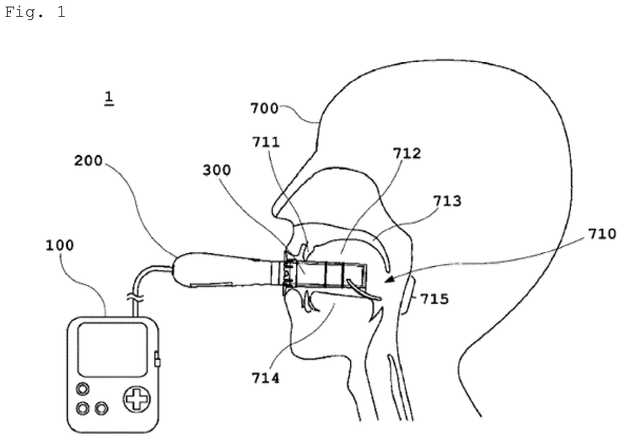

Fig. 1 is a diagram illustrating a usage state of the processing system 1 according to one embodiment of the present disclosure. According to Fig. 1, the processing system 1 according to the present disclosure includes a processing device 100 and a capturing device 200. A user inserts an aid 300 to an oral cavity 710 of the target, and inserts the capturing device 200 inside the aid to be covered with the inserted aid 300. Therefore, the capturing device 200 is used for capturing the inside of the oral cavity 710. Specifically, first, the user (may be a target 700 oneself, or may be a person other than the target 700) inserts the aid 300 for the capturing device 200 of the processing system 1 into the oral cavity 710. In this case, the head of the aid 300 is inserted to the vicinity of a soft palate 713 through incisor teeth 711. Then, in a state where the aid 300 is inserted into the oral cavity 710, the capturing device 200 is inserted into the aid 300 from the head. Therefore, the capturing device 200 is inserted to the vicinity of the soft palate 713 through the incisor teeth 711. In this case, a tongue 714 is pushed downward by the aid 300 (functioning as a tongue spatula) such that the movement of the tongue 714 is restricted, and the soft palate 713 is pushed upward by the head of the aid 300. Accordingly, an excellent visual field of the capturing device 200 is ensured, and a pharynx 715 positioned in front of the capturing device 200 is captured.

The captured subject image (the image of the pharynx 715) is transmitted to the processing device 100 connected to the capturing device 200 by wire such that communication is available. A processor of the processing device 100 that receives the subject image processes a program stored in a memory of the processing device 100, and thus, a region is specified on the basis of the subject image, and a result thereof is output to a display or the like.

Note that, as illustrated in Fig. 1, the head portion of the capturing device 200 is inserted to the vicinity of the pharynx of the target. Accordingly, the head portion is affected by the aspiration of the target, and is strongly affected by the clouding of the head portion, light diffusion, or the like, due to the clouding. Accordingly, the image to be obtained may be in a state of being clouded in white. In addition, as illustrated in Fig. 1, the capturing device 200 passes through the vicinity of the incisor teeth 711, and is inserted to the vicinity of the tongue 714. Accordingly, stable capturing may be disturbed by the movement of the incisor teeth 711 or the tongue 714. In addition, there are not only the pharynx 715 but also various regions such as the soft palate 713 or the tongue 714 in the vicinity of the pharynx 715, and thus, it may be difficult to adjust a focal point. Accordingly, the image to be obtained may be unsharp. Accordingly, the processing system 1 is capable of executing sharpening processing such as clouding removal processing or super-resolution processing.

### 2. Configuration of Processing System 1

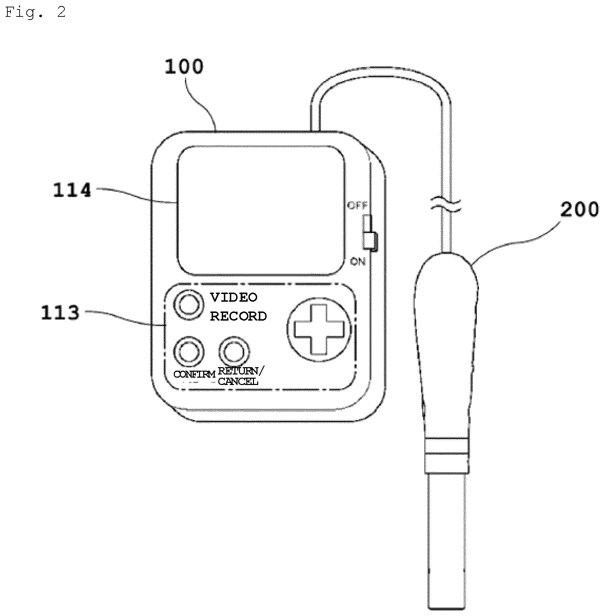

Fig. 2 is a schematic view of the processing system 1 according to one embodiment of the present disclosure. According to Fig. 2, the processing system 1 includes the processing device 100, and the capturing device 200 connected to the processing device 100 by wire such that communication is available. The processing device 100 receives manipulation input of the user, and controls the capturing of the capturing device 200. In addition, the processing device 100 processes the subject image captured by the capturing device 200, and specifies the region of the pharynx or the follicle included in the image. Further, the processing device 100 outputs the specified region to be identifiable such that the user, the target, or the like is capable of checking the region.

At least the head of the capturing device 200 is inserted into the oral cavity of the target, and the oral cavity, in particular, the pharynx is captured. Such specific capturing processing will be described below. The captured subject image is transmitted to the processing device 100 through a wired cable.

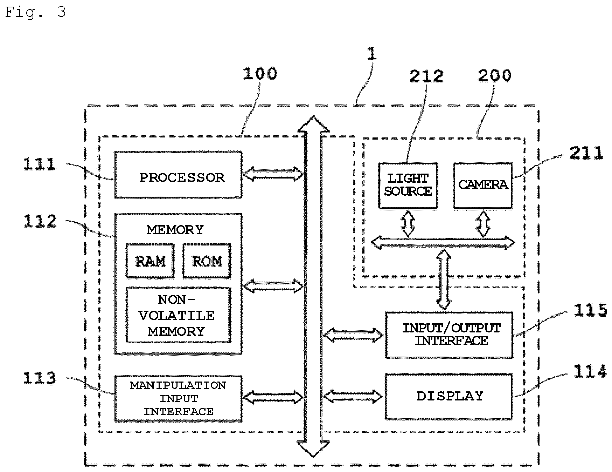

Fig. 3 is a block diagram illustrating the configuration of the processing system 1 according to one embodiment of the present disclosure. According to Fig. 3, the processing system 1 includes the processing device 100 including a processor 111, a memory 112, a manipulation input interface 113, a display 114, and an input/output interface 115, and the capturing device 200 including a camera 211 and a light source 212. Such constituents are electrically connected to each other through a control line and a data line. Note that, it is not necessary that the processing system 1 includes all the constituents illustrated in Fig. 3, the processing system can be configured by omitting a part of the constituents, and other constituents can also be added. For example, the processing system 1 can be provided with a battery for driving each of the constituents, a communication interface for transmitting a result processed by the processing system 1 to the outside or receiving a command from the outside, and the like.

The processor 111 functions as a control unit controlling other constituents of the processing system 1, on the basis of a program stored in the memory 112. The processor 111 controls the driving of the camera 211 and the driving of the light source 212, on the basis of the program stored in the memory 112, stores the subject image received from the capturing device 200 in the memory 112, and processes the stored subject image. Specifically, the processor 111 executes processing of receiving instruction input of the user to the manipulation input interface 113 and of turning on the light source 212 to instruct the camera 211 to capture an image, processing for receiving the input of the subject image captured by the capturing device 200, processing for storing the subject image received by the input/output interface 115 in the memory 112, processing for specifying a predetermined region in the subject included in the subject image, on the basis of the subject image stored in the memory 112, processing for processing the subject image such that the specified predetermined region is identifiable, processing for sharpening the subject image, processing for outputting the subject image processed such that the specified specific region is identifiable to the display 114, and the like, on the basis of the program stored in the memory 112. The processor 111 mainly includes one or a plurality of CPUs, and may be suitably combined with a GPU or the like.

The memory 112 includes a RAM, a ROM, a non-volatile memory, an HDD, and the like, and functions as a storage unit. The memory 112 stores a command for various control of the processing system 1 according to this embodiment as a program. Specifically, the memory 112 stores a program for the processor 111 to execute the processing of receiving the instruction input of the user to the manipulation input interface 113 and of turning on the light source 212 to instruct the camera 211 to capture an image, the processing for receiving the input of the subject image captured by the capturing device 200, the processing for storing the subject image received by the input/output interface 115 in the memory 112, the processing for specifying the predetermined region in the subject included in the subject image, on the basis of the subject image stored in the memory 112, the processing for processing the subject image such that the specified predetermined region is identifiable, the processing for sharpening the subject image, the processing for outputting the subject image processed such that specified specific region is identifiable to the display 114, and the like. In addition, the memory 112 stores various information relevant to a target including the target 700 (target identification information and the like), or stores the subject image captured by the camera 211 of the capturing device 200, the subject image subjected to sharpening processing or region specifying processing, and the like in association with the target identification information, in addition to the program. In addition, in some cases, the memory 112 stores each learned model that is used in the sharpening processing or the region specifying processing. Note that, as the memory 112, storage media connected to the outside such that communication is available can be used, or a combination of such storage media can also be used.

The manipulation input interface 113 functions as a manipulation input unit receiving the instruction input of the user to the processing device 100 and the capturing device 200. As illustrated in Fig. 2, examples of the manipulation input interface 113 include a "capture button" for instructing the capturing device 200 to start/end video recording, a "confirm button" for performing various selections, a "return/cancel button" for returning to the previous screen or canceling a confirmation manipulation that is input, an arrow key button for moving an icon or the like displayed on the display 114, an on/off key for turning on/off a power source of the processing device 100, and the like. Note that, as the manipulation input interface 113, a touch panel that is provided to be superimposed on the display 114 and includes an input coordinate system corresponding to a display coordinate system of the display 114. A detection method of the instruction input of the user by the touch panel may be any method such as a capacitive method and a resistive method.

The display 114 functions as a display unit for displaying the subject image captured by the capturing device 200 or for outputting a result determined by the processor 111. The display includes a liquid crystal panel, but is not limited to the liquid crystal panel, and may include an organic EL display, a plasma display, or the like.

The input/output interface 115 functions as an input/output unit for receiving various commands relevant to capturing start of the capturing device 200 connected in a wired manner or a wireless manner, or the input/output of image data captured by the capturing device 200. Examples of the input/output interface 115 include various interfaces such as a wired communication connector such as a USB or a SCSI, a wireless communication transceiver such as Bluetooth (Registered Trademark) or an infrared ray, and various connection terminals for a printed circuit board or a flexible printed circuit board. For example, in the example of Fig. 2, a wired cable extending from the capturing device 200 is connected to the input/output interface 115 that is a USB terminal. The image data of the subject image received by the input/output interface 115 is stored in the memory 112 under the control of the processor 111.

The camera 211 functions as a capturing unit that is driven in accordance with an instruction from the processing device 100, and detects the reflected light reflected on the oral cavity that is the subject to generate the subject image. In order to detect the light, as an example, the camera 211 includes a CMOS image sensor, and a lens system and a driving system for attaining a desired function. The image sensor is not limited to the CMOS image sensor, and other sensors such as a CCD image sensor can also be used as the image sensor. Even though it is not particularly illustrated, the camera 211 may have an automatic focus function, and for example, it is preferable that a focal point is set on the front surface of the lens to correspond with the specified region. In addition, the camera 211 may have a zoom function, and it is preferable that the capturing is set at a suitable magnification in accordance with the size of the pharynx or the influenza follicle.

In this embodiment, the camera 211 is inserted into the oral cavity of the target, and is used for capturing the pharynx at the back of the oral cavity, and thus, a distance between the camera 211 and the subject is comparatively short. Therefore, the camera 211 has a field angle (2θ) in which a value calculated by [(Distance from Tip Portion of Camera 211 to Posterior Wall of Pharynx)*tanθ] is 20 mm or more in a vertical way and is 40 mm or more in a horizontal way. By using the camera having such a field angle, it is possible to perform the capturing in a wider range even in a case where the distance between the camera 211 and the subject is short. Therefore, as the camera 211, a general camera can be used, and a camera referred to as a so-called wide-angle camera or super-wide-angle camera can also be used.

In addition, in this embodiment, the influenza follicle captured by the camera 211 is formed in the pharynx in the oral cavity. In general, since the pharynx is formed inside in a depth direction, in a case where the depth of field is shallow, the focal point is misaligned between the anterior portion of the pharynx and the posterior portion of the pharynx, and it is difficult to obtain a suitable subject image used in the determination of the processing device 100 or the medical doctor. Therefore, the camera 211 has a depth of field of at least 20 mm or more, preferably 30 mm or more. By using the camera having such a depth of field, it is possible to obtain an in-focus subject image in any region between the anterior portion of the pharynx and the posterior portion of the pharynx.

The light source 212 functions as a light source unit that is driven in accordance with the instruction from the processing device 100 and is for emitting light to the oral cavity. The light source 212 includes one or a plurality of light sources. In this embodiment, the light source 212 includes one or a plurality of LEDs, and light having a predetermined frequency band is emitted in the direction of the oral cavity from each of the LEDs. In the light source 212, light having a desired band from an ultraviolet light band, a visible light band, and an infrared light band, or a combination thereof is used. In particular, by emitting light having a short wavelength band in the ultraviolet light band to the influenza follicle, a specific component of the influenza follicle reacts, and thus, it is possible to more reliably determine the possibility of a disease.

Note that, in this embodiment, a case has been described in which the processing device 100 and the capturing device 200 are connected by the wired cable such that communication is available. However, naturally, the present disclosure is not limited thereto, the processing device and the capturing device may be connected by wireless communication.

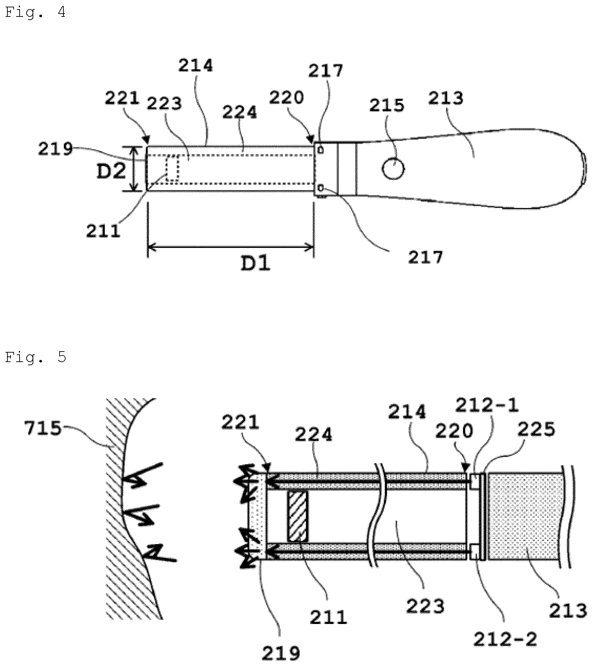

Fig. 4 is a schematic view illustrating the configuration of the top surface of the capturing device 200 according to one embodiment of the present disclosure. Hereinafter, a specific configuration of the capturing device 200 will be described on the basis of Fig. 4. According to Fig. 4, a main body 214 includes a base 220 and a head 221, and includes a columnar body having a predetermined length in a direction approximately parallel to a direction in which light is emitted from the light source 212. Then, at least the head 221 of the main body 214 is inserted to the oral cavity.

The main body 214 is formed to be columnar in the shape of a hollow cylinder with a perfectly circular sectional surface. A wall portion 224 thereof may contain any material insofar as light can be guided to the inside thereof, and as an example, a thermoplastic resin can be used. As the thermoplastic resin, a polyolefin-based resin such as a chain polyolefin-based resin (a polypropylene-based resin and the like) and a cyclic polyolefin-based resin (a norbornene-based resin and the like), a cellulose ester-based resin such as triacetyl cellulose and diacetyl cellulose, a polyester-based resin, a polycarbonate-based resin, a (meth)acrylic resin, a polystyrene-based resin, or a mixture or a copolymer thereof, and the like can be used. That is, the main body 214 functions as a light guide body for guiding light emitted from the light source in the oral cavity or in the direction of a diffusion plate.

Since the main body 214 is formed into a hollow shape, a housing space 223 is formed on the inner surface thereof by the wall portion 224. The camera 211 is housed in the housing space 223. Note that, it may be sufficient that the main body 214 is formed into the shape of a column including the housing space 223. Therefore, it is not necessary that the housing space 223 is in the shape of a cylinder with a perfectly circular sectional surface, and the sectional surface may be in the shape of an ellipse or a polygon. In addition, it is not necessary that the inside of the main body 214 is formed into a hollow shape.

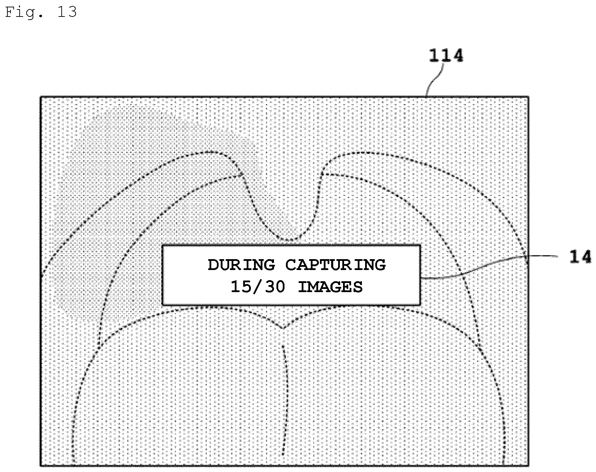

Here, as an example, the length of the main body 214 is set in a positional relationship with the incisor teeth of the target. Fig. 13 is a schematic view illustrating the sectional surface of the target 7 according to one embodiment of the present disclosure. Specifically, the sectional surface of the vicinity of the oral cavity of the general target 7 is illustrated. According to Fig. 13, in the target 7, the oral cavity is formed toward the direction of the back of the larynx from the incisor teeth 711, and the pharynx 715 that is the subject is in the deepest portion thereof. Therefore, in order to capture the pharynx 715, the head of the capturing device 200 is inserted to the vicinity of the soft palate 713. The target 7 has a distance d1 from the incisor teeth 711 to the soft palate 713. According to "Measurement of Inner Diameter of Upper Respiratory Tract in Sleep Apnea Syndrome (Nobuo OTANI)" J. Jpn. Bronchoesophagol. Soc. (The Journal of the Japan Broncho-esophagological Society), Vol. 40, No. 5, pp 396-402, in general, the distance d1 is approximately 100 mm to 200 mm.

The main body 214 has a distance D1 as a length from the head 221 to the base 220. The distance D1 may be a length of 100% or less of the distance d1 from the incisor teeth 711 to the soft palate 713, preferably a length of 80% or less. In general, in a case where foreign substances are inserted to the back of the larynx, a feeling of vomiting is caused, and in a case where the main body 214 is short, the distance between the camera 211 and the subject excessively increases. With the distance D1 described above, it is possible to prevent the feeling of vomiting, and it is possible to suitably retain a feeling of distance from the subject.

The head of a grip 213 is connected to the base 220 of the main body 214. The user grips the grip 213 and performs a manipulation such as the insertion and removal of the capturing device 200. The grip 213 is relatively narrowed toward the head side connected to the main body 214, in accordance with a shape when gripped with the palm of the user, and has a shape that is bent to swell toward the base side positioned on a side opposite to the main body 214. Note that, in this embodiment, the grip has a shape with a perfectly circular sectional surface, but it is not necessary to have a perfectly circular shape, and the shape may be an ellipse or a polygon.

Here, as an example, the width (a distance D2) of the main body 214 in a direction vertical to a direction connecting the head 221 and the base 220 of the main body 214 is set in a relationship with an opening width in an up-and-down direction of the mouth of the target. According to Fig. 13, the target 7 has a distance d2 as the opening width in the up-and-down direction of the mouth. According to "Statistical Examination for Maximum Opening Amount in Japanese Adults with Healthy Temporomandibular Joint (Hiroyasu TSUKAHARA et al.)" Japanese Journal of Oral and Maxillofacial Surgery, Vo. 44, No. 2, pp 159-167, the distance d2 is 3.5 cm to 4.0 cm on average in the case of general male adults.

The capturing device 200 is inserted at the width of the distance d2 together with the aid 300, and it is necessary for the user to capture the oral cavity while observing the oral cavity from a gap to which the capturing device is inserted. Accordingly, it is favorable not to hinder the visibility of the user in a state where the capturing device 200 is inserted. Therefore, the distance D2 of the main body 214 may be a width of 80% or less, preferably 60% or less of the distance d2 that is the opening width in the up-and-down direction of the mouth, or may be 3.2 cm or less, preferably 2.4 cm or less.

The grip 213 includes an engaging protrusion 217 for positioning the aid 300, and a positioning protrusion 218, in the vicinity of the base 220 of the main body 214. The engaging protrusion 217 engages with an engaging protrusion provided on the aid 300. In addition, the positioning protrusion 218 is inserted to an insert hole provided in the aid 300, and positions the capturing device 200 and the aid 300 to each other. Note that, in this embodiment, as the engaging protrusion 217 of the main body 214, a total of four engaging protrusions are arranged at an equal interval at a position in the vicinity of the base 220 of the main body 214 on the surface of the grip 213. In addition, one positioning protrusion 218 is arranged at a position in the vicinity of the base 220 of the main body 214 between the engaging protrusions 217 on the surface of the grip 213. However, the present disclosure is not limited thereto, and only one of the engaging protrusion 217 and the positioning protrusion 218 may be arranged. In addition, the number of engaging protrusions 217 or positioning protrusions 218 is not limited insofar as there are one or a plurality of engaging protrusions or positioning protrusions.

The grip 213 includes a capture button 215 for starting or ending the capturing of the subject image by receiving the manipulation input from the user. Therefore, the user is capable of instructing the start and the end of the capturing by using the capture button of the manipulation input interface 113 of the processing device 100, and is also capable of instructing the start and the end of the capturing by using the capture button 215.

A diffusion plate 219 is arranged on the head 221 of the main body 214, and diffuses light that is emitted from the light source 212 and passes through the main body 214 toward the inside of the oral cavity. The diffusion plate 219 has a shape corresponding to a sectional shape of a portion of the main body 214 that is configured to be capable of guiding light. In this embodiment, the main body 214 is formed into the shape of a hollow cylinder. Therefore, the sectional surface of the diffusion plate 219 is also formed into a hollow shape corresponding to the shape of the main body.

The camera 211 is used for detecting reflected light that is diffused from the diffusion plate 219, is emitted into the oral cavity, and is reflected on the subject, to generate the subject image. The camera 211 is arranged on the inner surface of the wall portion 224 of the main body 214, that is, in the housing space 223 formed inside the main body 214. Note that, in this embodiment, only one camera 211 is described, but the capturing device 200 may include a plurality of cameras. By generating the subject image using the plurality of cameras, the subject image includes information relevant to a steric shape of the influenza follicle. Accordingly, more accurately, it is possible to specify the region of the influenza follicle. In addition, in this embodiment, the camera 211 is arranged in the housing space 223 of the main body 214, and may be arranged on the head 221 of the main body 214 or in the main body 214 (may be inside the main body 214 or may be on the outer circumference of the main body 214).

Fig. 5 is a schematic view illustrating the configuration of the sectional surface of the capturing device 200 according to one embodiment of the present disclosure. According to Fig. 5, as the light source 212, a total of four light sources 212-1 to 212-4 are arranged on a substrate 225 arranged on the head side of the grip 213. As an example, each of the light sources 212 includes a LED, and light having a predetermined frequency band is emitted toward the direction of the oral cavity from each of the LEDs. Specifically, the light emitted from the light source 212 is incident on the base 220 of the main body 214, and is guided in the direction of the diffusion plate 219 by the wall portion 224 of the main body 214. The light that reaches the diffusion plate 219 is diffused by the diffusion plate 219 into the oral cavity. Then, the light diffused by the diffusion plate 219 is reflected on the pharynx 715 that is the subject, or the like. The reflected light reaches the camera 211, and thus, the subject image is generated.

Note that, the light sources 212-1 to 212-4 may be configured to be independently controlled. For example, by turning on a part of the light sources 212-1 to 212-4, the shade of the influenza follicle having a steric shape can be included in the subject image. Accordingly, it is possible to include the information relevant to the steric shape of the influenza follicle in the subject image, more accurately, it is possible to specify the region of the influenza follicle.

In addition, in this embodiment, the light sources 212-1 to 212-4 are arranged on the base 220 side of the main body 214, and may be arranged on the head 221 of the main body 214 or in the main body 214 (may be inside the main body 214 or may be on the outer circumference of the main body 214).

The diffusion plate 219 is used for preventing the light emitted from the light source 212 from lighting only a part of the oral cavity to generate homogeneous light. Therefore, as an example, a fine lens array is formed on the surface of the diffusion plate 219, and a lens-shaped diffusion plate having an arbitrary diffusion angle is used. In addition, as a modification, a diffusion plate that is capable of diffusing light by other methods, such as a diffusion plate that attains a light diffusion function by fine irregularities randomly arranged on the surface, may be used. Further, the diffusion plate 219 may be integrated with the main body 214. For example, the integration can be attained by a method of forming fine irregularities in the head portion of the main body 214.

Note that, in the capturing device, at least the head 221 of the main body 214 is inserted into the oral cavity. Therefore, the capturing of the subject is affected by the clouding of the head portion due to the aspiration of the target. Accordingly, in the processing device 100, the sharpening processing for removing the influence of the clouding can be implemented.

### 3. Processing Flow Executed in Processing Device 100

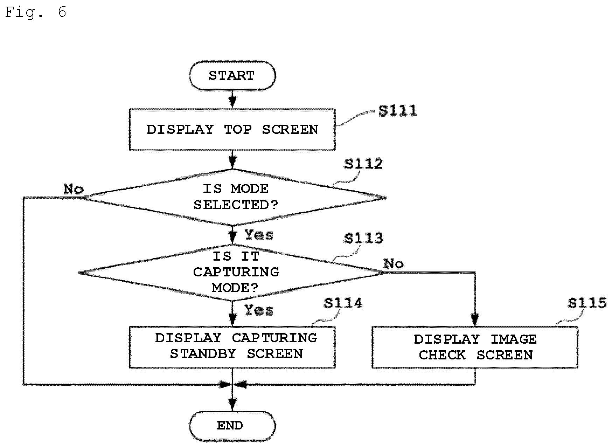

Fig. 6 is a diagram illustrating a processing flow that is executed in the processing device 100 according to one embodiment of the present disclosure. Specifically, Fig. 6 is a diagram illustrating the processing flow that is executed by the processor 111 of the processing device 100 in a predetermined cycle, on the basis of the program stored in the memory 112.

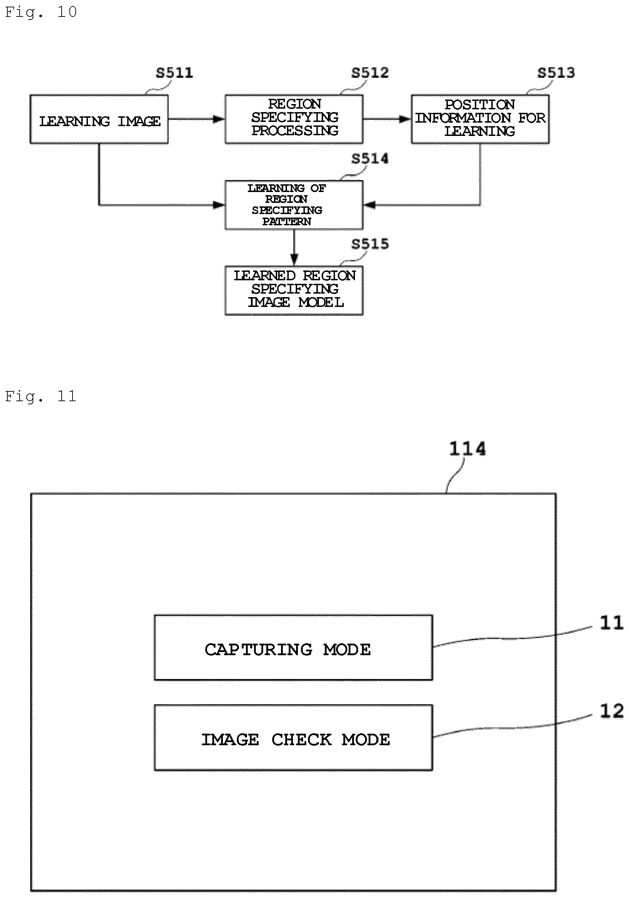

In a case where the processing flow is started, the processor 111 performs control such that a top screen is displayed on the display 114 (S111). Here, Fig. 11 is a diagram illustrating an example of a screen that is displayed on the processing device 100 according to one embodiment of the present disclosure. Specifically, an example of the top screen that is displayed on the display 114 by the processing of Sill in Fig. 6 is illustrated. According to Fig. 11, a capturing mode icon 11 for turning on the power source of the capturing device 200 to transition to a capturing mode of capturing the subject image, and an image check mode icon 12 for transitioning to an image check mode of outputting the subject image or the like that is previously captured and is stored in the memory 112 or the like to the display 114 are displayed approximately in the vicinity of the center of the display 114. The user is capable of manipulating the manipulation input interface 113 to select which mode to transition to.

Returning again to Fig. 6, the processor 111 determines whether the manipulation input of the user with respect to either of the capturing mode icon 11 or the image check mode icon 12 is received through the manipulation input interface 113 (S112). Then, in a case where it is determined that the capturing mode icon 11 is selected (S113), the processor 111 transitions to the capturing mode, and performs control such that the power source of the capturing device 200 is turned on and a capturing standby screen is output to the display 114 (S114). The capturing standby screen will be described below. On the other hand, in a case where it is determined that the image check mode icon 12 is selected (S113), the processor 111 transitions to the image check mode, and performs control such that an image check screen is output to the display 114 (S115). Accordingly, the processing flow is ended. Note that, even in a case where the processor 111 determines that no manipulation input is received in S112, the processing flow is ended as it is.

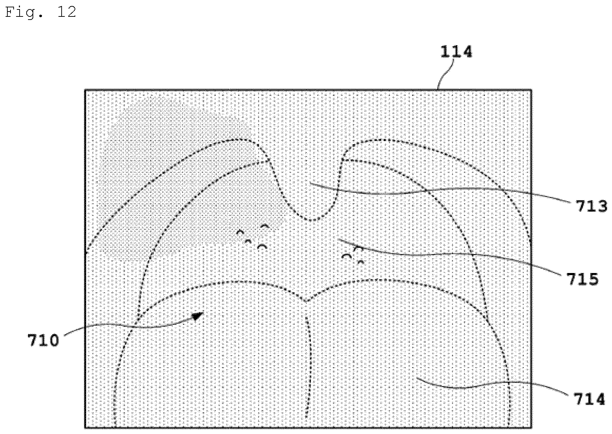

Here, Fig. 12 is a diagram illustrating an example of the screen that is displayed on the processing device 100 according to one embodiment of the present disclosure. Specifically, an example of the capturing standby screen that is displayed on the display 114 the processing of S114 in Fig. 6 is illustrated. According to Fig. 12, as the capturing standby screen, a so-called through image that is captured by the capturing device 200 and is received through the input/output interface 115 is displayed on the display 114. The user is capable of performing the manipulation input with respect to the manipulation input interface 113 to adjust a focal point, a field angle, an aperture value, and the like or to perform scaling or the like, with reference to the through image.

The user inserts at least the head portion of the capturing device 200 into the oral cavity of the target, and the subject image of the vicinity of the pharynx that is the subject is displayed on the capturing standby screen in Fig. 12. Accordingly, a subject image including the pharynx 715 in addition to the tongue 714 in the oral cavity 710 into which the capturing device 200 is inserted, and the soft palate 713 is displayed in the subject image. Here, as described above, in the capturing of the oral cavity 710, an image in which the subject image is entirely clouded in white or is locally (for example, the upper left portion of the image in Fig. 12) clouded in white due to the clouding of the head portion of the capturing device 200 that is caused by the influence of the expired air of the target, diffused reflection of the light from the light source that is caused by the moisture, local light emission, and the like.

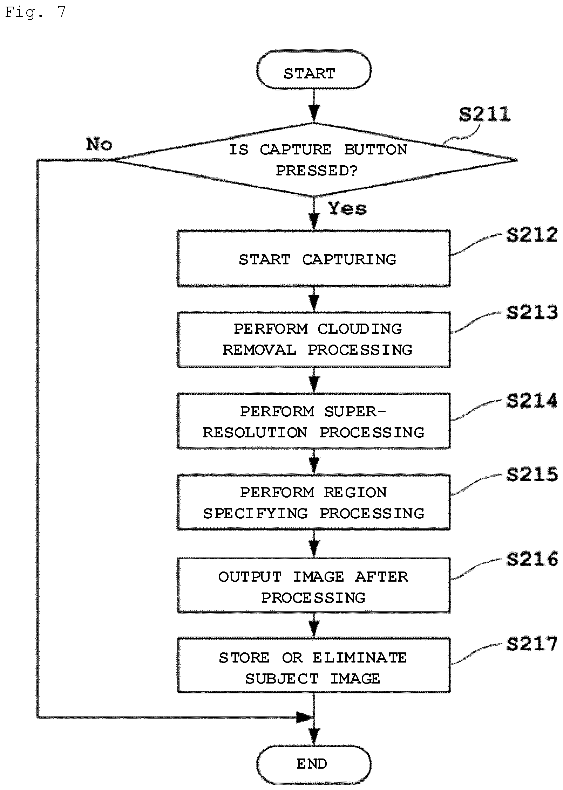

Fig. 7 is a diagram illustrating a processing flow that is executed in the processing device 100 according to one embodiment of the present disclosure. Specifically, Fig. 7 is a diagram illustrating the processing flow that is executed by the processor 111 of the processing device 100 in a predetermined cycle, on the basis of the program stored in the memory 112, when the capturing standby screen is displayed in Fig. 6.

The processing flow of Fig. 7 is started by the user selecting the capturing mode such that the capturing standby screen is displayed, as described above. In a case where the processing flow is started, the processor 111 determines whether any capture button is pressed by receiving the input from the manipulation input interface 113 or the capture button 215 of the capturing device 200 (S211). Then, in a case where any capture button is pressed, the capturing of the capturing device 200 is started (S212), and a screen during capturing is displayed. Specifically, the processor 111 performs control such that the subject image captured by the camera 211 of the capturing device 200 is input to the processing device 100 through the input/output interface in the capturing device 200 and the input/output interface 115 in the processing device 100, and is stored in the memory 112.

Note that, here, the subject image that is captured may be a still image in which one or a plurality of images are continuously captured each time when any capture button is pressed, or may be a moving image that is captured for a predetermined amount of time. In addition, even though it is not particularly illustrated, while a set of processing is executed in the processing device 100, the manipulation input of the user is received in the manipulation input interface 113, and the target to be the test subject is input in advance. Therefore, when the captured subject image is stored in the memory 112, the processor 111 stores the subject image in association with information such as the target identification information or a capturing date of the input target.

Here, Fig. 13 is a diagram illustrating an example of the screen that is displayed on the processing device 100 according to one embodiment of the present disclosure. Specifically, Fig. 13 illustrates an example of the screen that is displayed on the display 114 after the capturing is started by the processing of S212 in Fig. 7. According to Fig. 13, in a case where the capture button is pressed, a predetermined number of (for example, 30) images are continuously captured at a regular interval as a whole, and the current capturing progress is displayed on the display 14 (a display indicating that the 15th image of 30 images is being captured is displayed together with a display of "during capturing"). Then, the subject image that is captured by the camera 211 of the capturing device 200 at this point is displayed on the back surface of the display 14.

Returning again to Fig. 7, in a case where the capturing of the subject image is ended and each of the captured subject images is stored in the memory 112, the sharpening processing is performed with respect to each of the subject images. In the sharpening processing, various processing such as removal processing for various noise components such as clouding included in the subject image, edge portion emphasis processing, super-resolution processing, correction processing such as luminance correction, and binarization processing can be included. In Fig. 7, since the subject image is obtained by capturing the inside of the oral cavity, the subjected image is likely to be an image clouded in white due to the clouding by the expired air. Accordingly, the processor 111 performs the clouding removal processing with respect to the subject image stored in the memory 112 (S213). The processor 111 stores the subject image subjected to the clouding removal processing once in the memory 112, in association with the target identification information.

As an example of the clouding removal processing, the processor 111 executes processing such as the correction of a pixel value, and contrast and/or luminance of each pixel, and the application of a clouding removing filter with respect to the obtained subject image. In addition, by applying the obtained subject image to a learned clouding removal image model generated by learning in which a subject image including clouding is set to a clouding learning image, and a subject image not including clouding is set to an excellent learning image, it is also possible to implement the clouding removal processing.

Here, for example, the captured subject image may be enlargedly displayed in order to check a lesion region in detail. However, the subject image may be unsharp due to the degradation or the like of the obtained image, and thus, it may be difficult to sufficiently check the lesion region. Accordingly, the processor 111 performs the super-resolution processing with respect to the subject image subjected to the clouding removal processing (S214). The processor 111 stores the subject image subjected to the super-resolution processing once in the memory 112, in association with the target identification information.

Examples of the super-resolution processing include single-image super-resolution processing using a self correlation of an image, multiple-image super-resolution processing of estimating a fine positional misalignment between a plurality of images to perform interpolation between the pixels, and the like. In addition, by applying the obtained subject image to a learned super-resolution image model generated by learning in which a high-resolution image is set to a high-resolution learning image, and an image obtained by performing degradation processing with respect to the high-resolution learning image is set to a low-resolution learning image, it is also possible to implement the super-resolution processing.

Note that, in this embodiment, the clouding removal processing and the super-resolution processing the processing are performed in this order, but the processing order is not limited only thereto. For example, the order can also be reverse, and other processing can also be implemented by being suitably combined.

Next, the processor 111 performs the region specifying processing with respect to the subject image subjected to the super-resolution processing (S215). Then, the processor 111 stores each of the subject image before the region specifying processing that is stored in the memory 112 and the subject image that is subjected to the region specifying processing and is labeled such that the specified region is identifiable in the memory 112, in association with the target identification information.

Examples of the region specifying processing include labeling processing by the medical doctor or the like through the manipulation input interface 113, processing of retaining a feature amount database stored for each region specified in advance and of labeling on the basis of a feature amount in the obtained subject image, and the like. In addition, by applying the obtained subject image to a learned region specifying image model generated by learning in which the obtained subject image is set as a learning image, and position information of each region that is obtained by performing the region specifying processing with respect to the learning image is set to position information for learning, it is also possible to implement the region specifying processing. Note that, in this embodiment, a case is described in which the region of the pharynx and the follicle is mainly specified, but the same processing is performed even in a case where the tonsil or other regions are specified.

Next, the processor 111 performs processing for outputting each of the subject images subjected to the processing described above to the display 114 (S216). Note that, the display on the display 114 is an example of output processing, and other output methods are also available. As another example of the output, it is also possible to output to an external display device or a printer connected in a wired/wireless manner, or it is also possible to transmit to other terminal devices or a server device connected in a wired/wireless manner.

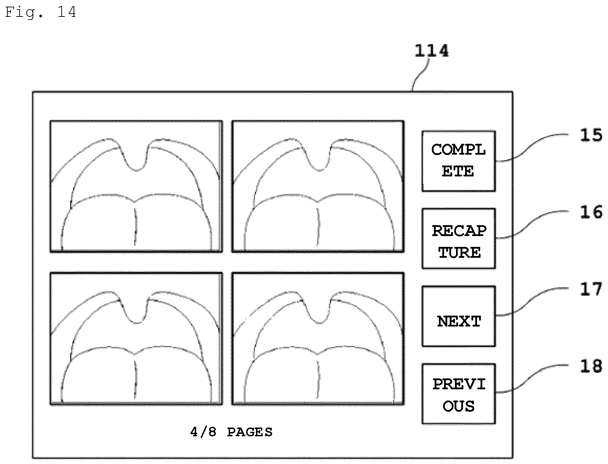

Here, Fig. 14 is a diagram illustrating an example of the screen that is displayed on the processing device 100 according to one embodiment of the present disclosure. Specifically, Fig. 14 illustrates an example of a list screen that is displayed on the display 114 after various processing is ended by the processing of S216 in Fig. 7. According to Fig. 14, 30 subject images obtained by pressing the capture button are displayed on the display 114 four by four in the capturing order thereof. In addition, a complete icon 15, a recapture icon 16, a next icon 17, and a previous icon 18 are each displayed on the right side of the display 114. The complete icon 15 is used for storing each of the subject image temporarily stored in the memory 112 in an HDD, a flash memory, or the like of the memory 112, in association with the target identification information, and for transitioning to a state of standing by until the press of the capture button is detected again. The recapture icon 16 is used for eliminating each of the subject images temporarily stored in the memory 112 from the memory 112, and then, for transitioning to a state of standing by until the press of the capture button is detected. The next icon 17 and the previous icon 18 are used for allowing a set of subject images displayed four by four to a state in which the next four subject images or the previous four subject images are displayed. Each of the icons is selected by receiving the manipulation input of the user in the manipulation input interface 113.

Note that, as also illustrated in Fig. 14, the subject images that have been subjected to the clouding removal processing (S213) or the super-resolution processing (S214) in Fig. 7 are displayed on the list screen. Therefore, each of the images is a high-resolution image from which clouding is removed. Not only a high-resolution image but also a low-resolution image can be displayed on the list screen.

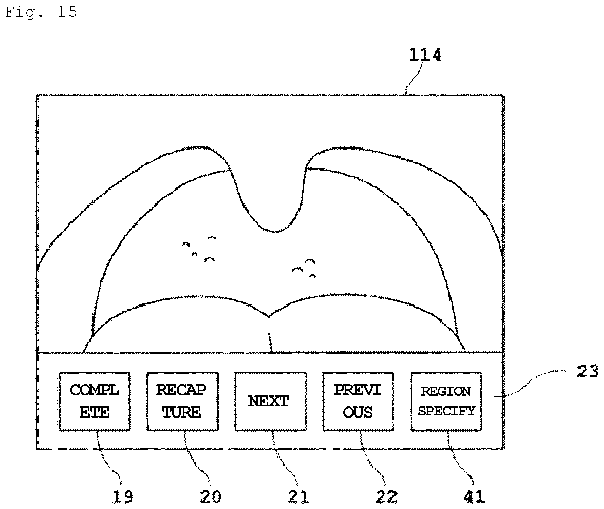

Fig. 15 is a diagram illustrating an example of the screen that is displayed on the processing device 100 according to one embodiment of the present disclosure. Specifically, Fig. 15 illustrates an example of the screen when one specific subject image selected by the user from the list screen of Fig. 14 is displayed on the display 114. The subject image on the list screen of Fig. 14 is selected by receiving the selection manipulation of the user through the manipulation input interface 113.

According to Fig. 15, one specific subject image selected by receiving the selection manipulation of the user, which has been subjected to the super-resolution processing and the clouding removal processing, is displayed on the display 114. In addition, the manipulation input interface 113 receives the manipulation input from the user, and then, a selection tray 23 is temporarily displayed to be superimposed on the subject image, in the lower portion of the display 114. The complete icon 19, the recapture icon 20, the next icon 21, and the previous icon 22 are each displayed in the selection tray 23. The function of each of the icons is the same as that of each of the icons described in Fig. 14. In addition, a region specifying icon 41 is displayed in the selection tray 23, in addition to the icons. The region specifying icon 41 is used for reading out the subject image that is labeled such that the specified region is identifiable from the memory 112 to display, corresponding to the subject image that is currently displayed. Note that, by the manipulation input interface 113 receiving the manipulation of the user on the list screen, the list screen is returned to the screen illustrated in Fig. 14.

As illustrated in Fig. 15, the subject images that have been subjected to the clouding removal processing (S213) or the super-resolution processing (S214) in Fig. 7 are displayed on the screen. In addition, by the manipulation input interface 113 receiving the input of a scaling manipulation of the user, the processor 111 is capable of scaling the displayed image. Therefore, even in a case where the image is enlarged, it is possible to display a high-resolution subject image.

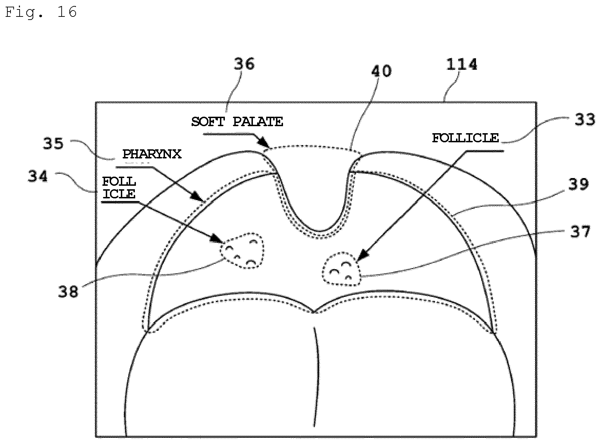

Fig. 16 is a diagram illustrating an example of the screen that is displayed on the processing device 100 according to one embodiment of the present disclosure. Specifically, Fig. 16 illustrates an example of the screen that is displayed by receiving the selection of the region specifying icon 41 by the user on the screen of Fig. 15. According to Fig. 16, label information for making each of the regions specified by the region specifying processing (S215) in Fig. 7 identifiable is displayed to be superimposed on the subject image. For example, a region specified as the soft palate is highlighted by a region display 40, and a region name display 36 notifying that it is the "soft palate" is performed. Similarly, a region specified as the pharynx is highlighted by a region display 39, and a region name display 35 notifying that it is the "pharynx" is performed. Further, a region specified as the influenza follicle is highlighted by region displays 37 and 38, and region name displays 33 and 34 notifying that it is the "follicle" are performed. As described above, by outputting each of the regions specified by the region specifying processing to the display 114 to be identifiable, it is possible for the medical doctor or the like to perform a smooth diagnosis. Note that, by the manipulation input interface 113 receiving the manipulation of the user on the screen, the screen returns to the screen illustrated in Fig. 15. Note that, in the example of Fig. 16, an example of specifying the region such as the pharynx, the soft palate, or the follicle is described, but the same processing is performed even in the case of specifying other regions such as the tonsil.

Returning again to Fig. 7, the processor 111 performs processing of storing each of the subject images temporarily stored in the memory 112 in the HDD, the flash memory, or the like of the memory 112, in association with the target identification information, or of eliminating the subject image without storing, on the basis of the manipulation input of the user that is received by the manipulation input interface 113 on each of the screens (S217). Then, the processing flow is ended.

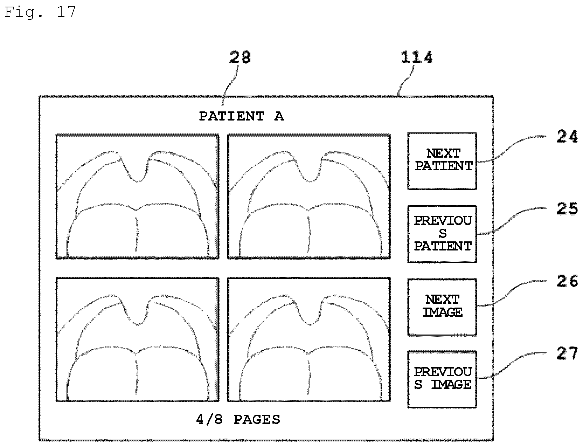

Here, Fig. 17 is a diagram illustrating an example of the screen that is displayed on the processing device 100 according to one embodiment of the present disclosure. Specifically, in S115 of Fig. 6, an example in which the mode transitions to the image check mode and the image check screen is displayed on the display 114 is illustrated. According to Fig. 17, a list screen of subject images of a target with the latest capturing order is displayed. Such images are read out by extracting a set of images associated with target identification information from the memory 112. Therefore, a target name (Patient: A) 28 that is specified by the target identification information associated with the displayed subject image is displayed in the upper portion of the screen. In addition, a next patient icon 24, a previous patient icon 25, a next image icon 26, and a previous image icon 27 are each displayed on the right side of the display 114. The next patient icon 24 and the previous patient icon 25 are used for displaying a set of subject images stored in association with targets stored before and after a target that is currently displayed. The next image icon 26 and the previous image icon 27 are used for allowing the set of subject images displayed four by four to transition to a state in which next four subject images or previous four subject images are displayed. Each of the icons is selected by receiving the manipulation input of the user in the manipulation input interface 113.

Note that, as also illustrated in Fig. 17, the subject images stored after being subjected to the clouding removal processing or the super-resolution processing are displayed on the list screen. Not only a high-resolution image but also a low-resolution image can be displayed on the list screen.

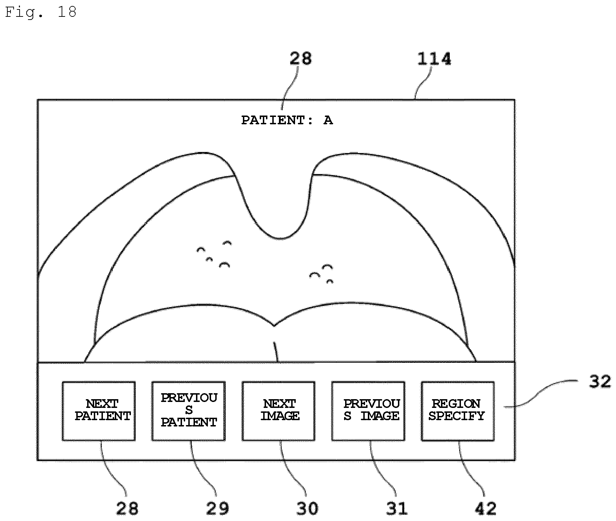

Fig. 18 is a diagram illustrating an example of the screen that is displayed on the processing device 100 according to one embodiment of the present disclosure. Specifically, Fig. 18 illustrates an example of the screen when one specific subject image selected by the user from the list screen illustrated in Fig. 17 is displayed on the display 114. The subject image is selected by receiving the selection manipulation of the user through the manipulation input interface 113 on the list screen of Fig. 17.

According to Fig. 18, one specific subject image selected by receiving the selection manipulation of the user is displayed on the display 114. In addition, the manipulation input interface 113 receives the manipulation input of the user, and then, a selection tray 32 is temporarily displayed to be superimposed on the subject image, in the lower portion of the display 114. The next patient icon 28, the previous patient icon 29, the next image icon 30, and the previous image icon 31 are each displayed in the selection tray 32. The function of each of the icons is the same as that of each of the icons described in Fig. 17. In addition, a region specifying icon 42 is displayed in the selection tray 32, in addition to the icons. The region specifying icon 41 is used for reading out the subject image that is labeled such that the specified region is identifiable from the memory 112 to display, corresponding to the subject image that is currently displayed. Note that, by the manipulation input interface 113 receiving the manipulation of the user on the list screen, the list screen is returned to the screen illustrated in Fig. 14.

As also illustrated in Fig. 18, the subject images stored after being subjected to the clouding removal processing or the super-resolution processing are displayed on the screen. In addition, by the manipulation input interface 113 receiving the input of a scaling manipulation of the user, the processor 111 is capable of scaling the displayed image. Therefore, even in a case where the image is enlarged, it is possible to display a high-resolution subject image.

Note that, in a case where the selection manipulation input with respect to the region specifying icon 42 is received, the subject image labeled such that the specified region is identifiable is displayed on the display 114. Since the screen is the same as that in Fig. 16, the description thereof will be omitted.

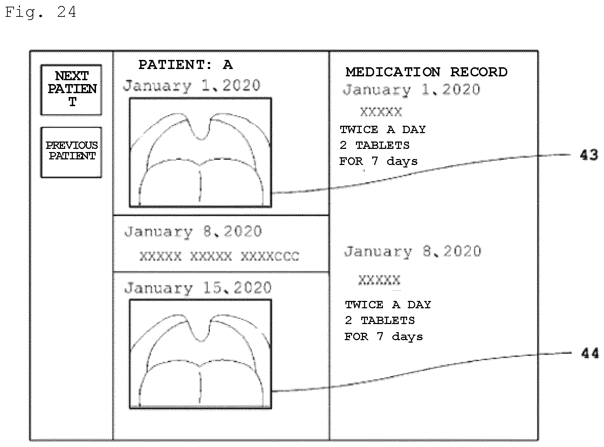

Fig. 24 is a diagram illustrating an example of image output according to one embodiment of the present disclosure. Specifically, Fig. 24 illustrates an example in which various information stored in association with the subject image is output to other processing devices connected in a wired manner or a wireless manner from the input/output interface 115. In the example of Fig. 24, in the other processing device, a capturing date, information of the medical opinion of the medical doctor, a registration record, and the like are stored for each target identification information piece, in addition to the subject image, and are output as an electronic medical chart. Then, according to Fig. 24, a capturing date and subject images 43 and 44 captured on the date are displayed on a display of the other processing device to be browsable by the medical doctor or the like, and the information of the medical opinion of the medical doctor, medication record information, and the like are displayed in addition to the subject image.

### 4. Processing Flow relevant to Generation of Learned Image Model

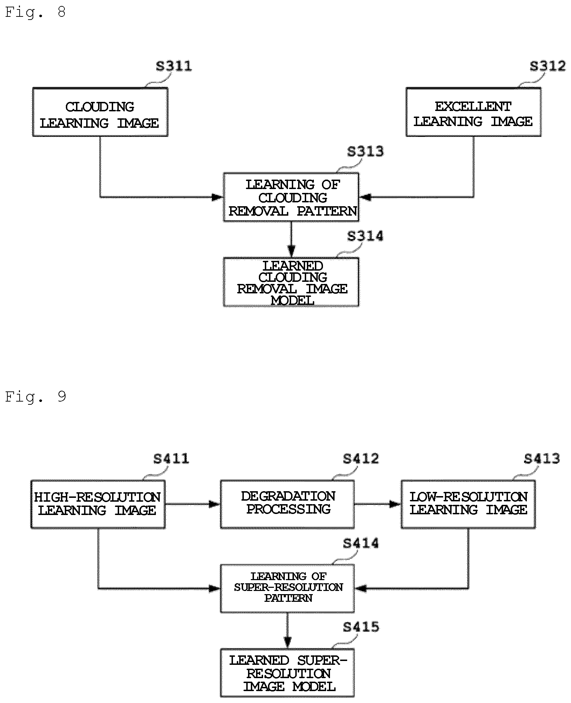

Fig. 8 is a diagram illustrating a processing flow relevant to the generation of a learned model according to one embodiment of the present disclosure. Specifically, Fig. 8 is processing for generating the learned clouding removal image model that is used in S213 of Fig. 7. The processing flow may be executed by the processor 111 of the processing device 100, or may be executed by a processor of the other processing device.

According to Fig. 8, a step of acquiring the subject image including clouding, which is captured by the capturing device 200, as the clouding learning image is executed (S311). A step of acquiring an excellent subject image not including clouding, which is separately captured by the capturing device 200, as the excellent learning image is executed (S312). Note that, the excellent learning image is acquired by using the subject image that is captured separately from the clouding learning image, and may be acquired by performing the clouding removal processing such as the correction of a pixel value, and contrast and/or luminance of each pixel, and the application of a clouding removing filter with respect to the clouding learning image.

In a case where each of the clouding learning image and the excellent learning image is obtained, a step of performing machine learning of a clouding removal pattern is executed (S313). As an example of the machine learning, a set of the clouding learning image and the excellent learning image are applied to a neural network configured in combination with neurons, and the learning is repeated while adjusting a parameter of each of the neurons such that the output of the neural network is the same as the excellent learning image. Then, a step of acquiring the learned clouding removal image model (for example, the neural network and the parameter) is executed (S314). The acquired learned clouding removal model may be stored in the memory 112 of the processing device 100 or the other processing device connected to the processing device 100 through wireless communication. Then, by executing the stored learned clouding removal model in the processing device 100 or the other processing device, the clouding removal processing (S213) of the subject image illustrated in Fig. 7 is executed.

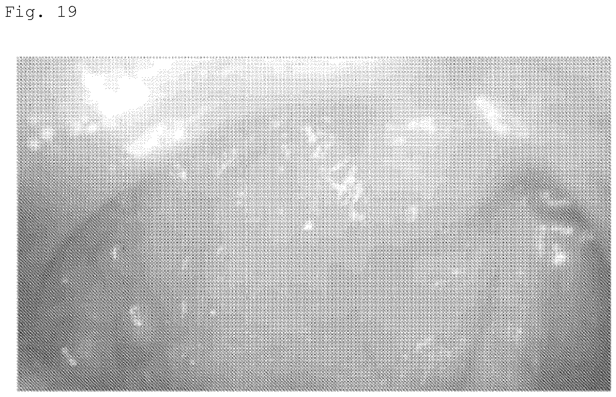

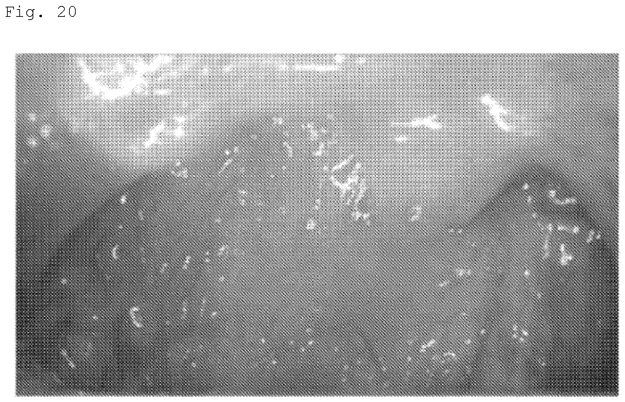

Fig. 19 and Fig. 20 are diagrams illustrating an example of the image obtained by the processing device 100 according to one embodiment of the present disclosure. Specifically, Fig. 19 illustrates a subject image captured by the capturing device 200, before the clouding removal processing. In addition, Fig. 20 illustrates a subject image obtained after performing the clouding removal processing with respect to the image in Fig. 19 using the learned clouding removal model. According to Fig. 19, the subject image before the clouding removal processing is an image of which all or a part is clouded in white by the expired air of the target, the diffused reflection of light due to the moisture contained in the expired air, and light concentration on a specific portion. Accordingly, in the image, the visibility in the vicinity of the pharynx of the oral cavity is degraded. On the other hand, according to Fig. 20, by performing the clouding removal processing using the learned clouding removal model with respect to the image in Fig. 19, the clouding is removed, and the visibility of the soft palate or the pharynx at the back thereof is obviously improved.

Note that, in order to obtain the subject image after the clouding removal processing in Fig. 20, the learned clouding removal model is generated by applying 100 clouding learning images and 100 excellent learning images to the neural network and by performing the machine learning.

Fig. 9 is a diagram illustrating a processing flow relevant to the generation of the learned model according to one embodiment of the present disclosure. Specifically, Fig. 9 is processing for generating the learned super-resolution image model that is used in S214 of Fig. 7. The processing flow may be executed by the processor 111 of the processing device 100, or may be executed by a processor of the other processing device.

According to Fig. 9, a step of acquiring the high-resolution image of the subject including the pharynx as the high-resolution learning image is executed (S411). Next, a step of performing the degradation processing such as scale down processing and feathering processing with respect to the acquired high-resolution learning image is executed (S412). Then, a step of acquiring an image after the degradation processing, which is obtained as a result of the execution, as the low-resolution learning image is executed (S413).

In a case where each of the high-resolution learning image and the low-resolution learning image is obtained, a step of performing machine learning of a super-resolution pattern by using both the learning images is executed (S414). As an example of the machine learning, a set of the high-resolution learning image and the low-resolution learning image are applied to a neural network configured in combination with neurons, and the learning is repeated while adjusting a parameter of each of the neurons such that the output of the neural network is the same as the high-resolution learning image. Then, a step of acquiring the learned super-resolution image model (for example, the neural network and the parameter) is executed (S415). The acquired learned super-resolution image model may be stored in the memory 112 of the processing device 100 or the other processing device connected to the processing device 100 through wireless communication. Then, by executing the stored learned super-resolution image model in the processing device 100 or the other processing device, the super-resolution processing (S214) of the subject image illustrated in Fig. 7 is executed.

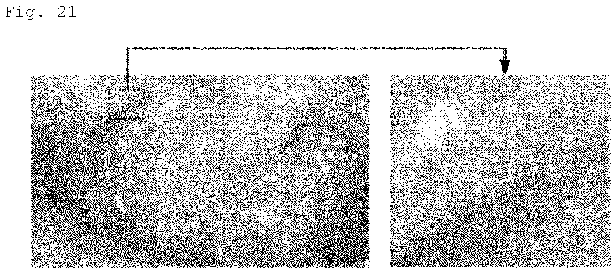

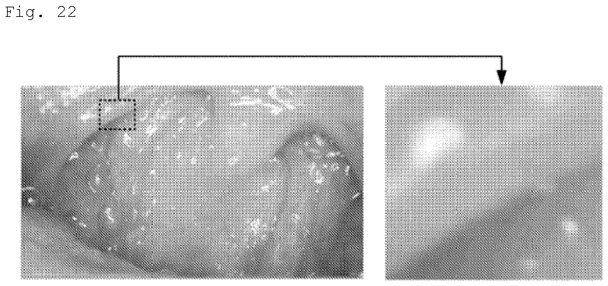

Fig. 21 and Fig. 22 are diagrams illustrating an example of the image obtained by the processing device 100 according to one embodiment of the present disclosure. Specifically, Fig. 21 is a diagram illustrating a subject image captured by the capturing device 200, before the super-resolution processing, and a partially enlarged image thereof. In addition, Fig. 22 is a subject image obtained after performing the super-resolution processing with respect to the image in Fig. 21 using the learned super-resolution image model, and a partially enlarged image thereof. According to Fig. 21, in the subject image before the super-resolution processing, as it is also obvious from the partially enlarged image thereof, a boundary portion of each of the regions is unsharp. On the other hand, according to Fig. 22, by performing the super-resolution processing using the learned super-resolution image model with respect to the image in Fig. 21, as it is also obvious from the partially enlarged image thereof, the unsharpness of the boundary portion of each of the regions is eliminated, and the sharpness is improved to the extent that the boundary can be sharply grasped even when the image is enlarged.

Note that, in order to obtain the subject image after the super-resolution processing in Fig. 22, the learned super-resolution image model is generated by applying 500 high-resolution learning images and 500 low-resolution learning images obtained by performing the degradation processing with respect to the image to the neural network and by performing the machine learning.

Fig. 10 is a diagram illustrating a processing flow relevant to the generation of the learned model according to one embodiment of the present disclosure. Specifically, Fig. 10 is processing for generating the learned region specifying image model that is used in S215 of Fig. 7. The processing flow may be executed by the processor 111 of the processing device 100, or may be executed by a processor of the other processing device.

According to Fig. 10, a step of acquiring the subject image of the subject including the pharynx as the learning image is executed (S511). Next, a step of performing the labeling processing for an attention region in the diagnosis of the pharynx, the follicle, or the like with respect to the acquired learning image, for example, on the basis of the manipulation input of the medical doctor, is executed (S512). Then, a step of acquiring position information of a label obtained as a result of the execution as the position information for learning is executed (S513).

In a case where each of the learning image and the position information for learning is obtained, a step of performing machine learning of a region specifying pattern using the learning image and the position information is executed (S514). As an example of the machine learning, a set of the learning image and the position information for learning are applied to a neural network configured in combination with neurons, and the learning is repeated while adjusting a parameter of each of the neurons such that the output of the neural network is the same as the position information for learning. Then, a step of acquiring the learned region specifying image model (for example, the neural network and the parameter) is executed (S515). The acquired learned region specifying image model may be stored in the memory 112 of the processing device 100 or the other processing device connected to the processing device 100 through wireless communication. Then, by executing the stored learned region specifying image model in the processing device 100 or the other processing device, the region specifying processing (S215) of the subject image illustrated in Fig. 7 is executed.

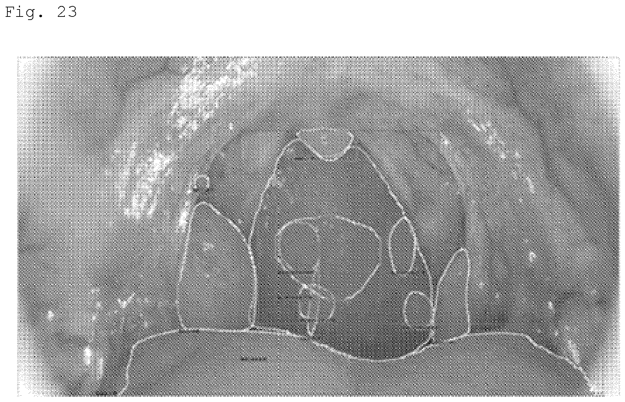

Fig. 23 is a diagram illustrating an example of the image obtained by the processing device 100 according to one embodiment of the present disclosure. Specifically, Fig. 23 illustrates a subject image that is output such that each of the specified regions is identifiable by performing the region specifying processing using the learned region specifying image model, after performing the clouding removal processing and the super-resolution processing with respect to the subject image captured by the capturing device 200. According to Fig. 23, by using the learned region specify model, it is possible to specify each region such as the pharynx or the follicle. In addition, in the example of Fig. 10, examples of the attention region include only the pharynx and the follicle, and it is also possible to specify a part such as the tonsil or the tongue, or a region that is randomly generated, such as air bubbles or saliva.

Note that, in order to obtain the subject image after the region specifying processing in Fig. 23, the learned region specifying image model is generated by applying the position information for learning of 1300 learning images and each subject image obtained by performing the region specifying processing with respect to 1300 learning images to the neural network and by performing the machine learning.

As described above, in this embodiment, it is possible to provide the processing device, the processing program, the processing method, and the processing system that are suitable for processing the image obtained by capturing the oral cavity to be used in the diagnosis of the oral cavity. In addition, the region such as the pharynx or the tonsil is specified from the image obtained by capturing the oral cavity, and is output to be identifiable, which is preferable for the diagnosis of the medical doctor. In addition, in the capturing of the oral cavity that is easily affected by the expired air, or the movement of the teeth, the tongue, or the like, it is possible to improve the visibility by performing the clouding removal processing or the super-resolution processing.

### <Another Embodiment>

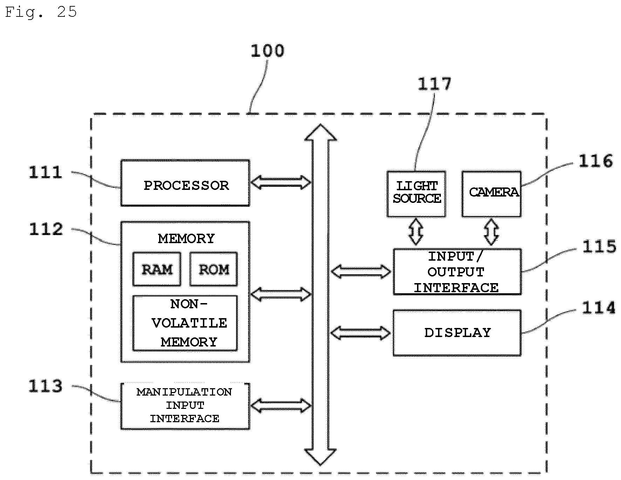

In the embodiment described above, a case has been described in which the processing device 100 and the capturing device 200 are connected by the wired cable such that communication is available. However, the present disclosure is not limited thereto, and the camera or the light source can also be integrally mounted on the flexible printed circuit board or the printed circuit board in the processing device 100. Fig. 25 is a block diagram illustrating the configuration of the processing system 1 according to another embodiment of the present disclosure. According to Fig. 25, as with one embodiment described above, the processing device 100 of the processing system 1 includes the processor 111, the memory 112, the manipulation input interface 113, the display 114, and the input/output interface 115. In addition, the processing device 100 further includes a camera 116 and a light source 117. Such constituents are electrically connected to each other through a control line and a data line, and the camera 116 or the light source 117 is also electrically connected to each of the constituents through the input/output interface 115.

Examples of the processing device 100 include a camera-equipped terminal device such as a smart phone, a mobile phone terminal, a tablet terminal, a PDA, a digital camera terminal, a portable game machine, and a laptop personal computer terminal. In this case, the camera 116 is arranged outside the incisor teeth (outside the body) without being inserted to the vicinity of the pharynx in the oral cavity, and captures the oral cavity.

In addition, in one embodiment described above, a case has been described in which the processing device 100 and the capturing device 200 are connected through the wired cable such that communication is available. However, the present disclosure is not limited thereto, and the processing device and the capturing device may be connected by wireless communication. In this case, for example, the subject image captured by the capturing device 200 is transmitted to a server device installed remotely, and the server device is allowed to function as the processing device, and thus, the subject image can be output to the display or the like of the other processing device.

Note that, in such an embodiment, the configuration, the processing, and the procedure are the same as those in one embodiment described in Fig. 1 to Fig. 22, except for the details described above. Therefore, the detailed description of the configuration, the processing, and the procedure will be omitted.

It is also possible to configure the system by suitably combining or replacing each of the constituents described in each of the embodiments.

The processing and the procedure described in this specification can be attained not only by those explicitly described in the embodiment but also by software, hardware, or a combination thereof. Specifically, the processing and the procedure described in this specification are attained by implementing logic corresponding to the processing in a medium such as an integrated circuit, a volatile memory, a non-volatile memory, a magnetic disk, and an optical storage. In addition, the processing and the procedure described in this specification can be executed in various computers including the processing device and the server device by being implemented as a computer program.

Even in a case where it is described that the processing and the procedure described in this specification are executed by a single device, single software, a single component, or a single module, such processing or procedure can be executed by a plurality of devices, a plurality of software pieces, a plurality of components, and/or a plurality of modules. In addition, even in a case where it is described that various information described in this specification is stored in a single memory or a single storage unit, such information can be dispersedly stored in a plurality of memories provided in a single device or a plurality of memories dispersedly arranged in a plurality of devices. Further, the constituents of the software and the hardware described in this specification can be attained by integrating the constituents to fewer constituents or by disintegrating the constituents to more constituents.

### Reference Signs List

- 1: processing system
- 100: processing device
- 200: capturing device
- 700: target

## Claims

1. A processing device, comprising:
an input/output interface connected to a camera capturing an image of a subject including at least an oral cavity such that communication is available and configured to receive input of the image captured by the camera;
a memory configured to store the image received by the input/output interface, in addition to computer readable instructions; and
a processor configured to execute the computer readable instructions stored in the memory so as to specify a predetermined region in the subject included in the image, on the basis of the image stored in the memory, and to process the image such that the specified predetermined region is identifiable.

2. The processing device according to claim 1,
wherein the predetermined region is specified on the basis of a program stored in the memory.

3. The processing device according to claim 1 or 2,
wherein the predetermined region is specified by a learned region specifying image model obtained by learning using a region learning image in which the subject including at least the oral cavity is captured and position information of the predetermined region that is included in the region learning image.

4. The processing device according to any one of claims 1 to 3,
wherein the processor is configured to perform sharpening processing for sharpening the image stored in the memory.

5. The processing device according to claim 4,
wherein the sharpening processing includes processing of removing clouding included in the image from the image.

6. The processing device according to claim 4 or 5,
wherein the sharpening processing is performed by a learned clouding removal image model obtained by learning using a clouding learning image in which clouding is included in the image and an excellent learning image in which clouding is not included in the image.

7. The processing device according to any one of claims 4 to 6,
wherein the sharpening processing includes super-resolution processing of increasing a resolution of the image.

8. The processing device according to any one of claims 4 to 7,
wherein the sharpening processing is performed by a learned super-resolution image model obtained by learning using a high-resolution learning image including the subject and a low-resolution learning image obtained by performing degradation processing with respect to the high-resolution learning image.

9. The processing device according to any one of claims 1 to 8,
wherein the predetermined region is a pharynx included in the oral cavity.

10. The processing device according to any one of claims 1 to 8,
wherein the predetermined region is a follicle formed in a pharynx included in the oral cavity.

11. The processing device according to any one of claims 1 to 8,
wherein the predetermined region is a tonsil included in the oral cavity.

12. The processing device according to any one of claims 1 to 11,
wherein the image processed by the processor such that the specified predetermined region is identifiable is output to another processing device through the input/output interface, and is output to be browsable as an electronic medical chart.

13. A processing device, comprising:
an input/output interface connected to a camera capturing an image of a subject including at least an oral cavity such that communication is available and configured to receive input of the image captured by the camera;
a memory configured to store the image received by the input/output interface, in addition to computer readable instructions; and
a processor configured to execute the computer readable instructions stored in the memory to perform processing of removing clouding included in the image from the image stored in the memory.

14. A processing device, comprising:
an input/output interface connected to a camera capturing an image of a subject including at least an oral cavity such that communication is available and configured to receive input of the image captured by the camera;
a memory configured to store the image received by the input/output interface, in addition to computer readable instructions; and
a processor configured to execute the computer readable instructions stored in the memory to perform super-resolution processing with respect to the image for increasing a resolution of the image stored in the memory.

15. A processing program allowing a computer, which includes: an input/output interface connected to a camera capturing an image of a subject including at least an oral cavity such that communication is available; and a memory configured to store the image, to function as
a processor configured to receive input of the image captured by the camera, to store the image received by the input/output interface, to specify a predetermined region in the subject included in the image, on the basis of the image stored in the memory, and to process the image such that the specified predetermined region is identifiable.

16. A processing method performed by executing computer readable instructions with a processor in a computer comprising an input/output interface connected to a camera capturing an image of a subject including at least an oral cavity such that communication is available; and a memory configured to store the image, in addition to the computer readable instructions, the method comprising the steps of:
receiving input of the image captured by the camera;
storing the image received by the input/output interface;
specifying a predetermined region in the subject included in the image, on the basis of the image stored in the memory; and
a processing the image such that the specified predetermined region is identifiable.

17. A processing system, comprising:
a capturing device configured to capture an image of a subject including at least an oral cavity; and
the processing device according to any one of claims 1 to 14.
